# EUROPÄISCHE PATENTSCHRIFT

(11) **EP 1 373 232 B1**
(45) Veröffentlichungstag und Bekanntmachung des Hinweises auf die Patenterteilung: **23.11.2005**
(21) Anmeldenummer: 02735133.7
(22) Anmeldetag: 15.03.2002
(51) Int. Cl.: C07D 295/02, A01N 43/84

(54) **VERFAHREN ZUR HERSTELLUNG VON N-SUBSTITUIERTEN 2,6-DIALKYLMORPHOLINEN**
METHOD FOR PRODUCING N-SUBSTITUTED 2,6-DIALKYL MORPHOLINES
PROCEDE POUR PREPARER DES 2,6-DIALKYLE MORPHOLINES N-SUBSTITUEES

(30) Priorität: 16.03.2001 DE 10112686
(43) Veröffentlichungstag der Anmeldung: 02.01.2004
(73) Patentinhaber: BASF Aktiengesellschaft, 67056 Ludwigshafen (DE)
(72) Erfinder: HENNINGSEN, Michael, 67227 Frankenthal (DE); KUSCHE, Andreas, 67483 Kleinfischlingen (DE); HÜLLMANN, Michael, 64625 Bensheim (DE); RÜB, Lothar, 67346 Speyer (DE); KÄSHAMMER, Stefan, 67105 Schifferstadt (DE); GERLACH, Till, 67071 Ludwigshafen (DE)
(74) Vertreter: Pohl, Michael Friedrich
(86) Internationale Anmeldenummer: PCT/EP2002/002916
(87) Internationale Veröffentlichungsnummer: WO 2002/074755

(56) Entgegenhaltungen:
- EP-A- 0 111 928
- EP-A- 0 129 211
- EP-A- 0 271 750
- DE-A- 2 118 283
- DE-A- 2 543 279
- DE-B- 1 014 547
- GB-A- 1 591 144
- LEO PAQUETTE: "Encyclopedia of reagents for organic synthesis" 1995 , WILEY , ENGLAND XP002202679 5 Platinum on Alumina p.4159-4160 Eq. 4 and platinum Oxide p.4162-4163 Eq. 1 & 2.
- HOUBEN-WEYL: METHODEN DER ORGANISCHEN CHEMIE, E15, TEIL 1: "En,X- und In,X-Verbindungen" 1993 , GEORG THIEME VERLAG , STUTTGART XP002202680 Herstellung von Enamine: p.598-612 und reduktive Hydrierung p.693

## Beschreibung

Die vorliegende Erfindung betrifft ein Verfahren zur Herstellung von N-substituierten 2,6-Dialkylmorpholinen durch Umsetzung einer Carbonylverbindung mit einem sekundären Amin in einer reduktiven Aminierung.

Tertiäre Amine bilden eine wichtige Verbindungsklasse, die äußerst vielfältige technische Anwendung findet. Daher besteht ein ständiger Bedarf an möglichst einfachen Verfahren, die die Herstellung tertiärer Amine aus möglichst gut zugängigen Komponenten in hohen Ausbeuten und mit hoher Selektivität gegenüber der angestrebten Zielverbindung ermöglichen. Von den heterocyclischen tertiären Aminen werden beispielsweise die N-substituierten Tetrahydro-1,4-oxazine (Morpholine) im Pflanzenschutz eingesetzt.

K. H. König et al. beschreiben in Angewandte Chemie 77 (1965), S. 327 - 333 N-substituierte Tetrahydro-1,4-oxazine und deren Einsatz als fungizide Verbindungen mit guter Wirkung gegen Pilzerkrankungen von Kulturpflanzen. Danach kann die Herstellung dieser Verbindungen beispielsweise durch dehydratisierende Cyclisierung der entsprechenden Bis-(2-hydroxyethyl)amine erfolgen.

Die DE-A-25 43 279 beschreibt ebenfalls ein Verfahren zur Herstellung von N-substituierten Tetrahydro-1,4-oxazinen durch ein- oder zweistufige Cyclisierung und Hydrierung von N-substituierten Bis-(2-hydroxyalkyl)aminen.

Nachteilig an dem zuvor genannten Verfahren ist die aufwendige Herstellung der eingesetzten Ausgangsstoffe.

Die DE-A-197 20 475 beschreibt ein Verfahren zur Herstellung von N-Alkyl-2,6-dialkylmorpholinen in einer zweistufigen Synthese, umfassend die Herstellung eines Oxazolidins durch Umsetzung entsprechender Aldehyde oder Ketone mit Dialkoholen sekundärer Amine in Gegenwart eines sauren Ionenaustauschers und die anschließende Umsetzung des Oxazolidins in Gegenwart von Wasserstoff und einem Hydrierkatalysator.

Die direkte Herstellung von tertiären Aminen aus Carbonylverbindungen und speziell aus Ketonen und sekundären Aminen gilt allgemein als schwierig, da die Zielverbindungen in der Regel nur in geringen Ausbeuten erhalten werden und ihre Isolierung dementsprechend aufwendig ist.

Die DE-AS 1 014 547 offenbart ein Verfahren zur reduktiven Alkylierung von Amino- und Nitroverbindungen mittels Ketonen oder Aldehyden und mit Wasserstoff. Als geeignete Aminoverbindungen werden unter Anderem Aminocyclohexane offenbart. Der Einsatz von Aminen, bei denen das Amin-Stickstoffatom Teil eines Heterocyclus ist, ist nicht offenbart.

Die GB-A-1 591 144 betrifft Morpholin-Derivate und deren Verwendung als Fungizide. Ihre Herstellung kann beispielsweise durch direkte Umsetzung von Terpenaldehyden oder Terpenketonen mit Morpholin-Verbindungen unter Hydrierungsbedingungen erfolgen. Gemäß Ausführungsbeispiel 13 erfolgt die Reduktion mit Wasserstoff in Gegenwart von Raney-Nickel.

Die DE-A-33 21 712 beschreibt 2,6-trans-Dimethylmorpholin-Derivate und deren Einsatz als Fungizide. Danach erfolgt z. B. die Herstellung von N-(Cyclododecyl)-2,6-dimethylmorpholin (Dodemorph) in einer zweistufigen Synthese, umfassend die Umsetzung von Cyclododecanon und 2,6-trans-Dimethylmorpholin in Gegenwart von p-Toluolsulfonsäure zu N-Cyclododecenyl-2,6-trans-dimethylmorpholin und dessen anschließende Hydrierung in Gegenwart eines Pd/C-Katalysators.

Die EP-A-0 271 750 beschreibt fungizide 4-substituierte Cyclohexylamine. Diese können z. B. durch reduktive Aminierung von Cyclohexanonen mit einem sekundären Amin in Gegenwart eines Reduktionsmittels erhalten werden. Als geeignete Reduktionsmittel werden Wasserstoff, Ameisensäure und komplexe Hydride, wie Natriumcyanoborhydrid genannt. Geeignete Katalysatoren für die reduktive Aminierung in Gegenwart von Wasserstoff werden nicht beschrieben. Diese Reaktionsvariante ist auch nicht durch ein Ausführungsbeispiel belegt.

Die DE-A-21 18 283 beschreibt ein Verfahren zur Herstellung sekundärer oder tertiärer aliphatischer oder cycloaliphatischer Amine durch Umsetzung einer aliphatischen oder cycloaliphatischen Carbonylverbindung mit Ammoniak oder einem primären bzw. sekundären Amin in Gegenwart von Wasserstoff und eines Hydrierkatalysators, wobei als Katalysator eine Mischung aus Silber und Palladium auf einem gesinterten Träger eingesetzt wird. Nachteilig an diesem Verfahren sind die hohen Kosten für den eingesetzten Silberkatalysator. Zudem sind die erhaltenen Ausbeuten und die Selektivitäten bezüglich der Zielverbindungen noch verbesserungswürdig.

Der vorliegenden Erfindung liegt die Aufgabe zugrunde, ein verbessertes Verfahren zur Herstellung von N-substituierten 2,6-Dialkylmorpholinen zur Verfügung zu stellen. Dabei soll die Synthese ausgehend von Carbonylverbindungen und sekundären Aminen in einer einstufigen Reaktion erfolgen. Vorzugsweise soll das Verfahren die Herstellung tertiärer Amine in hohen Ausbeuten und mit großer Selektivität gegenüber den angestrebten zielverbindungen ermöglichen.

Überraschenderweise wurde nun gefunden, dass diese Aufgabe durch ein Verfahren zur Herstellung von N-substituierten 2,6-Dialkylmorpholinen gelöst wird, bei dem man wenigstens eine Carbonylverbindung mit wenigstens einem Morpholinderivat in Gegenwart von Wasserstoff und wenigstens einem metallhaltigen Katalysator, dessen aktive Komponente im Wesentlichen aus Platingruppenmetallen besteht, in einer reduktiven Aminierung umsetzt.

Gegenstand der Erfindung ist somit ein Verfahren zur Herstellung von N-substituierten 2,6-Dialkylmorpholinen der allgemeinen Formel I 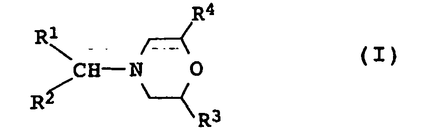 worin
- R¹ und R²: unabhängig voneinander für Wasserstoff, Alkyl oder Cycloalkyl stehen, oder R¹ und R² zusammen mit dem Kohlenstoffatom, an das sie gebunden sind, für einen 5- bis 14-gliedrigen Carbocyclus stehen, und
- R³ und R⁴: unabhängig voneinander für Alkyl oder Cycloalkyl stehen,
durch Umsetzung wenigstens einer Carbonylverbindung der allgemeinen Formel II 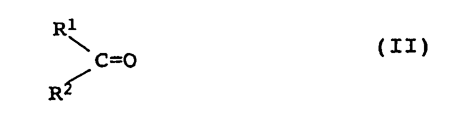 worin R¹ und R² die oben angegebenen Bedeutungen besitzen, mit wenigstens einem Morpholin der allgemeinen Formel III 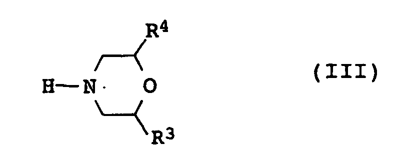 worin R³ und R⁴ die oben angegebenen Bedeutungen besitzen, in Gegenwart von Wasserstoff und wenigstens eines metallhaltigen Katalysators, das dadurch gekennzeichnet ist, dass die aktive Komponente des Katalysators im Wesentlichen aus Platingruppenmetallen besteht.

Im Rahmen der vorliegenden Erfindung umfasst der Ausdruck 'Alkyl' geradkettige und verzweigte Alkylgruppen. Vorzugsweise handelt es sich dabei um geradkettige oder verzweigte C₁-C₂₀-Alkyl-, bevorzugterweise C₁-C₁₂-Alkyl- und besonders bevorzugt C₁-C₈-Alkyl- und ganz besonders bevorzugt C₁-C₄-Alkylgruppen. Beispiele für Alkylgruppen sind insbesondere Methyl, Ethyl, Propyl, Isopropyl, n-Butyl, 2-Butyl, sec.-Butyl, tert.-Butyl, n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, n-Heptyl, 2-Heptyl, 3-Heptyl, 2-Ethylpentyl, 1-Propylbutyl, Octyl, Nonyl, Decyl.

Substituierte Alkylreste weisen vorzugsweise 1, 2, 3, 4 oder 5, insbesondere 1, 2 oder 3 Substituenten auf. Diese sind beispielsweise ausgewählt unter Cycloalkyl, Aryl, Hetaryl, Halogen, OH, SH, Alkoxy, Alkylthio, NE¹E², (NE¹E²E³)⁺, Carboxyl, Carboxylat, -SO₃H, Sulfonat, Nitro und Cyano.

Bei der Cycloalkylgruppe handelt es sich vorzugsweise um eine C₆-C₁₂-Cycloalkylgruppe, wie Cyclohexyl, Cycloheptyl, Cyclooctyl, Cyclodecyl oder Cyclododecyl. Besonders bevorzugt sind Cyclohexyl und Cyclododecyl.

Wenn die Cycloalkylgruppe substituiert ist, weist sie vorzugsweise 1, 2, 3, 4 oder 5, insbesondere 1, 2 oder 3 Substituenten auf. Diese sind beispielsweise ausgewählt unter Alkyl, Alkoxy, Alkylthio, OH, SH, Cycloalkyl, Cycloalkylalkyl, Nitro, Cyano oder Halogen.

Aryl steht vorzugsweise für Phenyl, Tolyl, Xylyl, Mesityl, Naphthyl, Anthracenyl, Phenanthrenyl, Naphthacenyl und insbesondere für Phenyl oder Naphthyl.

Substituierte Arylreste weisen vorzugsweise 1, 2, 3, 4 oder 5, insbesondere 1, 2 oder 3 Substituenten auf. Diese sind beispielsweise ausgewählt unter Alkyl, Alkoxy, Carboxyl, Carboxylat, Trifluormethyl, -SO₃H, Sulfonat, NE¹E², Alkylen-NE¹E², Nitro, Cyano oder Halogen.

Hetaryl steht vorzugsweise für Pyrrolyl, Pyrazolyl, Imidazolyl, Indolyl, Carbazolyl, Pyridyl, Chinolinyl, Acridinyl, Pyridazinyl, pyrimidinyl oder Pyrazinyl.

Substituierte Hetarylreste weisen vorzugsweise 1, 2 oder 3 Substituenten, ausgewählt unter Alkyl, Alkoxy, Carboxyl, Carboxylat, -SO₃H, Sulfonat, NE¹E², Alkylen-NE¹E², Trifluormethyl oder Halogen auf.

Die obigen Ausführungen zu Alkylresten gelten entsprechend für Alkoxy- und Alkylthioreste.

Die Reste NE¹E² stehen vorzugsweise für N,N-Dimethyl, N,N-Diethyl, N,N-Dipropyl, N,N-Diisopropyl, N,N-Di-n-butyl, N,N-Di-tert.-butyl, N,N-Dicyclohexyl oder N,N-Diphenyl.

Halogen-steht für Fluor, Chlor, Brom und Iod, bevorzugt für Fluor, Chlor und Brom.

Carboxylat und Sulfonat stehen im Rahmen dieser Erfindung vorzugsweise für ein Derivat einer Carbonsäurefunktion bzw. einer Sulfonsäurefunktion, insbesondere für ein Metallcarboxylat oder -sulfonat, eine Carbonsäure- oder Sulfonsäureesterfunktion oder eine Carbonsäure- oder Sulfonsäureamidfunktion.

Die aktive Komponente des erfindungsgemäß eingesetzten Katalysators besteht im Wesentlichen aus Platingruppenmetallen, d. h. Ru, Rh, Pd, Os, Ir, Pt und Gemischen davon.

Vorzugsweise wird ein Katalysator eingesetzt, dessen aktive Komponente im Wesentlichen frei von Silber ist.

Bevorzugt ist der Einsatz eines Katalysators, dessen aktive Komponente
- 1 bis 100 Gew.-%, bevorzugt 10 bis 99 Gew.-%, Pd,
- 0 bis 60 Gew.-%, vorzugsweise 1 bis 55 Gew.-%, Pt, und
- 0 bis 50 Gew.-%, wie z. B. 0,1 bis 40 Gew.-%, wenigstens eines weiteren Metalls, das insbesondere ausgewählt ist unter Ru, Rh, Os, Ir, Ce, La und Gemischen davon,
enthält.

Bevorzugt wird in dem erfindungsgemäßen Verfahren ein Katalysator eingesetzt, der einen Träger umfasst. Geeignete Träger sind ganz allgemein die üblichen, dem Fachmann bekannten Trägermaterialien. Dazu zählen beispielsweise kohlenstoffhaltige Trägermaterialien, wie Aktivkohle, Siliciumcarbid, polymere Träger, Metallträger, z. B. aus Edelstahl, Aluminiumoxide, Siliciumdioxide, Silikate, Alumosilikate, wie zeolithe, Bimsstein, Diatomeenerde, Kieselgel, Hydrotalcit, Titandioxide, Zirkoniumdioxide, Zinkoxid, Magnesiumoxid und Kombinationen und Gemische davon. Gegebenenfalls können die Trägermaterialien mit Alkali- und/oder Erdalkalimetalloxiden dotiert sein. Besonders bevorzugt werden α-Al₂O₃, γ-Al₂O₃, SiO₂, TiO₂, ZrO₂ und Gemische davon eingesetzt. Besonders bevorzugt ist ZrO₂. Die Träger können allgemein übliche Formen aufweisen und beispielsweise als Extrudate (in Form von Strängen), Pellets, Perlen, Tabletten, Ringe, Sättel, Gewebe, Gestricke, Monolithe, Kugeln, Pulver, etc. eingesetzt werden. Für ein diskontinuierliches Verfahren ist der Einsatz als Pulver bevorzugt.

Die eingesetzten Katalysatoren können nach allgemein bekannten Verfahren hergestellt werden, beispielsweise durch Tränken eines Trägers mit Lösungen von Verbindungen oder Komplexen der eingesetzten Metalle. Geeignete Metallverbindungen (Precursoren) sind beispielsweise Metallsalze, wie Nitrate, Nitrosylnitrate, Halogenide, Carbonate, Carboxylate, Metallkomplexe, wie Acetylacetonate, Halogenkomplexe, z. B. Chlorokomplexe, Aminkomplexe etc. Das Aufbringen der Metallverbindungen auf den Träger kann beispielsweise durch gemeinsames Fällen oder Tränken erfolgen. Werden mehrere Metallverbindungen eingesetzt, so können diese gleichzeitig oder nacheinander aufgebracht werden. Dabei ist die Reihenfolge, in der die aktiven Komponenten aufgebracht werden, in der Regel beliebig. Geeignete Lösungsmittel zur Herstellung der Katalysatoren durch Tränken sind Wasser und organische Lösungsmittel, wie Alkohole, z. B. Methanol und Ethanol, Aromaten, wie Benzol und Toluol, aliphatische Lösungsmittel, wie Hexan, Heptan, etc., cycloaliphatische Lösungsmittel, wie Cyclohexan, etc.

Die Herstellung von geträgerten palladiumhaltigen Katalysatoren erfolgt vorzugsweise durch Tränken eines Trägers mit einer Lösung von Pd(NO₃)₂, PdCl₂, H₂PdCl₄, Pd-Acetylacetonat, etc.

Nach dem Tränken wird der Träger vorzugsweise getrocknet. Dabei liegt die Temperatur im Allgemeinen in einem Bereich von etwa 50 bis 200 °C, bevorzugt 100 bis 150 °C. Nach dem Trocknen kann der Träger gewünschtenfalls calziniert werden. Dabei liegt die Temperatur im Allgemeinen in einem Bereich von 200 bis 600 °C, bevorzugt 400 bis 500 °C. Die Dauer der Calzinierung kann in einem weiten Bereich variieren und liegt z. B. bei etwa 1 bis 10 Stunden, bevorzugt 1,5 bis 5 Stunden. Zur Überführung der Precursoren in die aktive Komponente kann der Katalysator mit einem üblichen Reduktionsmittel wie Wasserstoff behandelt werden. Dabei können dem Reduktionsmittel gewünschtenfalls inerte Gase, wie Stickstoff oder Argon, beigemischt werden. Die Temperaturen bei der Reduktion liegen vorzugsweise in einem Bereich von etwa 100 bis 500 °C, besonders bevorzugt 200 bis 300 °C. Wird zur Herstellung der erfindungsgemäß eingesetzten Katalysatoren eine thermisch leicht zersetzbare Metallverbindung als Precursor eingesetzt, so werden diese in der Regel bereits unter den Calzinierbedingungen zu elementaren Metallen oder oxidischen Metallverbindungen zersetzt, so dass eine anschließende Reduktion im Allgemeinen nicht erforderlich ist.

An die Trocknung und/oder die Calzinierung und/oder die Reduktion kann sich noch wenigstens ein weiterer Behandlungsschritt anschließen. Dazu zählt beispielsweise die Passivierung z. B. mit Sauerstoff, dem gegebenenfalls wenigstens ein Inertgas beigemischt sein kann. Die Passivierung wird vorzugsweise zur Herstellung von Katalysatoren auf Basis von Metallen eingesetzt, bei denen auch die Metalloxide katalytische Aktivität aufweisen.

Weitere Verfahren zur Herstellung erfindungsgemäß einsetzbarer Katalysatoren sind dem Fachmann bekannt und umfassen z. B. Bedampfen, Sputtern, Ionenaustauschverfahren, etc.

Nach einer geeigneten Ausführungsform wird der Katalysator in situ mit Wasserstoff reduziert und so in die aktive Form überführt.

Die Oberfläche, das Porenvolumen und die Porengrößenverteilung des Katalysators sind in weiten Bereichen unkritisch.

Besonders bevorzugt wird in dem erfindungsgemäßen Verfahren ein Katalysator eingesetzt, der 0,1 bis 10 Gew.-% Pd, bezogen auf das Gewicht von aktiver Komponente und Träger, enthält. Vorzugsweise enthält der Katalysator 0 bis 5 Gew.-%, wie z. B. 0,1 bis 4 Gew.-%, Pt. Besonders bevorzugt sind Katalysatoren, die nur Pd als aktive Komponente enthalten.

Das erfindungsgemäße Verfahren ermöglicht in vorteilhafter Weise die einstufige Herstellung von N-substituierten 2,6-Dialkylmorpholinen. Dabei werden die Zielverbindungen in der Regel in hohen Ausbeuten und mit hoher Selektivität erhalten. Vorteilhafterweise ermöglicht das erfindungsgemäße Verfahren die Herstellung von N-substituierten 2,6-Dialkylmorpholinen auch bei hohen Eduktzulaufmengen, d. h. es werden im Allgemeinen gute Raum-Zeit-Ausbeuten erzielt. Ein weiterer Vorteil des erfindungsgemäßen Verfahrens liegt darin, dass es die isomerenreine Herstellung von N-substituierten 2,6-Dialkylmorpholinen ermöglicht.

Vorzugsweise wird zur Herstellung der N-substituierten 2,6-Dialkylmorpholine ein Keton der allgemeinen Formel II eingesetzt, worin R¹ und R² zusammen mit dem Kohlenstoffatom, an das sie gebunden sind, für einen 6- bis 12-gliedrigen Carbocyclus stehen, der einen, zwei oder drei Substituenten, die unabhängig voneinander ausgewählt sind unter Alkyl, Alkoxy, Alkylthio, Cycloalkyl und Cycloalkylalkyl, aufweisen kann. Besonders bevorzugt steht die Verbindung der Formel II für Cyclododecanon.

Vorzugsweise stehen die Reste R³ und R⁴ in der Formel III unabhängig voneinander für C₁-C₄-Alkylreste. Besonders bevorzugt stehen R³ und R⁴ beide für Methyl.

Das erfindungsgemäße Verfahren eignet sich insbesondere zur Herstellung von N-(Cyclododecyl)-2,6-dimethylmorpholin (Dodemorph).

Die Reaktionstemperatur liegt vorzugsweise in einem Bereich von 100 bis 300 °C.

Der Reaktionsdruck beträgt vorzugsweise 5 bis 300 bar, besonders bevorzugt 10 bis 250 bar.

Das erfindungsgemäße Verfahren kann lösungsmittelfrei oder in Gegenwart eines Lösungsmittels durchgeführt werden. Geeignete Lösungsmittel sind Wasser, Alkohole, wie Methanol und Ethanol, Ether, wie Methyl-tert.-butylether, cyclische Ether, wie Tetrahydrofuran, Ketone, wie Aceton und Methylethylketon, etc. Besonders bevorzugt wird das als Edukt eingesetzte Morpholin als Lösungsmittel eingesetzt. Dabei kann das Morpholin in einem bis zu 100-fachen molaren Überschuss gegenüber der Aminkomponente eingesetzt werden.

Das erfindungsgemäße Verfahren kann diskontinuierlich oder kontinuierlich durchgeführt werden. Bevorzugt ist die kontinuierliche Fahrweise.

Geeignete Reaktoren zur Durchführung des erfindungsgemäßen Verfahrens sind die üblichen, dem Fachmann bekannten Apparaturen zum Arbeiten unter erhöhtem Druck, wie z. B. Autoklaven oder Rohrreaktoren. Vorzugsweise erfolgt der Einsatz des Katalysators in Form eines Festbettes oder eines anderen geeigneten Einbaus. Vorzugsweise ist der Reaktionsraum senkrecht angeordnet.

Die Umsetzung erfolgt vorzugsweise in Rieselfahrweise oder in Sumpffahrweise. Dabei werden die Ausgangsstoffe bevorzugt so zugeführt, dass im Wesentlichen die gesamte Katalysatorschicht zusammenhängend mit Flüssigkeit bedeckt ist.

Die Erfindung wird anhand der folgenden Beispiele näher erläutert.

### Beispiele

### Beispiel 1 (Vergleich Pd/Ag-Katalysator):

In einen senkrecht stehenden Rohrreaktor wurden 500 ml eines Katalysators gefüllt, der 5 % Ag₂O und 0,4 % PdO auf einem SiO₂-Träger enthielt. Bei einer Temperatur von 220 °C und einem Wasserstoffdruck von 100 bar wurde von unten ein auf 220 °C erhitztes Gemisch aus einem Teil cis-2,6-Dimethylmorpholin, einem Teil trans-2,6-Dimethylmorpholin und 0,39 Teilen Cyclododecanon mit einer Zulaufmenge von 360 ml/h zugepumpt.

Das austretende Reaktionsgemisch enthielt, nach Entfernung des 2,6-Dimethylmorpholins im Vakuum, 89,6 % Dodemorph sowie 2,3 % Cyclododecanon. Dies entspricht einem Umsatz von 97,3 % und einer Selektivität von 91 %.

### Beispiel 2 (Pd/ZrO₂-Katalysator) :

Es wurde wie in Beispiel 1 verfahren, wobei jedoch reines cis-2,6-Dimethylmorpholin und ein Pd/ZrO₂-Katalysator (Palladiumgehalt 0,9 %) eingesetzt wurde. Bei gleicher Temperatur und Zulaufmenge und Abtrennung des 2,6-Dimethylmorpholins enthielt das Reaktionsgemisch 94,4 % Dodemorph sowie 0,7 % Cyclododecanon. Dies entspricht einem Umsatz von 99,3 % und einer Selektivität von 95 %.

### Beispiel 3 (Pd/ZrO₂-Katalysator):

Es wurde wie in Beispiel 2 verfahren, jedoch mit doppelter Zulaufmenge (720 ml/h) und einer Temperatur von 240 °C. Nach Abtrennung des 2,6-Dimethylmorpholins enthielt das Reaktionsgemisch 92,2 % Dodemorph sowie 2,6 % Cyclododecanon. Dies entspricht einem Umsatz von 97,4 % und einer Selektivität von 95 %.

### Beispiel 4 (Pd/Pt, ZrO₂-Katalysator):

Es wurde wie in Beispiel 1 verfahren, wobei jedoch reines cis-2,6-Dimethylmorpholin und ein Pd/Pt/ZrO₂-Katalysator (Palladiumgehalt 0,4 %, Platingehalt 0,4 %) eingesetzt wurde. Bei einer Temperatur von 230 °C und einer Zulaufmenge von 180 ml/h enthielt das Reaktionsgemisch nach Abtrennung des 2,6-Dimethylmorpholins 95,6 % cis-Dodemorph und kein Cyclododecanon. Dies entspricht einem Umsatz von 100 % und einer Selektivität von 95 %.

## Patentansprüche

1. Verfahren zur Herstellung von N-substituierten 2,6-Dialkylmorpholinen der allgemeinen Formel I 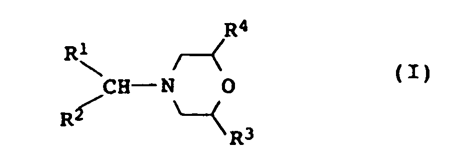 worin
R¹ und R² unabhängig voneinander für Wasserstoff, Alkyl oder Cycloalkyl stehen, oder R¹ und R² zusammen mit dem Kohlenstoffatom, an das sie gebunden sind, für einen 5- bis 14-gliedrigen Carbocyclus stehen, und
R³ und R⁴ unabhängig voneinander für Alkyl oder Cycloalkyl stehen,
durch Umsetzung wenigstens einer Carbonylverbindung der allgemeinen Formel II 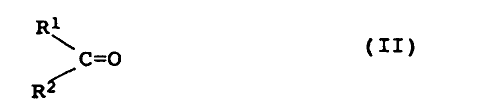 worin R¹ und R² die oben angegebenen Bedeutungen besitzen, mit wenigstens einem Morpholin der allgemeinen Formel III 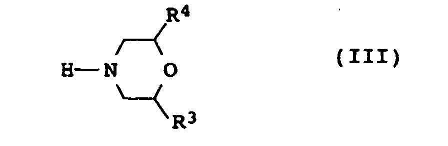 worin R³ und R⁴ die oben angegebenen Bedeutungen besitzen, in Gegenwart von Wasserstoff und wenigstens eines metallhaltigen Katalysators, **dadurch gekennzeichnet, dass** die aktive Komponente des Katalysators im Wesentlichen aus Platingruppenmetallen besteht.

2. Verfahren nach Anspruch 1, **dadurch gekennzeichnet, dass** man einen Katalysator einsetzt, dessen aktive Komponente
- 1 bis 100 Gew.-% Pd,
- 0 bis 60 Gew.-% Pt, und
- 0 bis 50 Gew.-% wenigstens eines weiteren Metalls, das ausgewählt ist unter Ru, Rh, Os, Ir, Ce, La und Gemischen davon,
enthält.

3. Verfahren nach einem der vorhergehenden Ansprüche, **dadurch gekennzeichnet, dass** man einen Katalysator einsetzt, der einen Träger umfasst.

4. Verfahren nach Anspruch 3, **dadurch gekennzeichnet, dass** man als Träger ZrO₂ einsetzt.

5. Verfahren nach einem der vorhergehenden Ansprüche, **dadurch gekennzeichnet, dass** man einen Katalysator einsetzt, der 0,1 bis 10 Gew.-% Pd und 0 bis 5 Gew.-% Pt enthält.

6. Verfahren nach einem der vorhergehenden Ansprüche, **dadurch gekennzeichnet, dass** R¹ und R² zusammen mit dem Kohlenstoffatom, an das sie gebunden sind, für einen 6- bis 12-gliedrigen Carbocyclus stehen, der einen, zwei oder drei Substituenten, die unabhängig voneinander ausgewählt sind unter Alkyl, Alkoxy, Alkylthio, Cycloalkyl und Cycloalkylalkyl, aufweisen kann.

7. Verfahren nach einem der vorhergehenden Ansprüche, **dadurch gekennzeichnet, dass** R³ und R⁴ unabhängig voneinander für C₁-C₄-Alkylreste stehen.

8. Verfahren nach einem der vorhergehenden Ansprüche, **dadurch gekennzeichnet, dass** R³ und R⁴ beide für Methyl stehen.

9. Verfahren nach einem der vorhergehenden Ansprüche, **dadurch gekennzeichnet, dass** es sich bei der Verbindung der allgemeinen Formel I um N-(Cyclododecyl)-2,6-dimethylmorpholin handelt.

## Claims

1. A process for the preparation of N-substituted 2,6-dialkylmorpholines of the formula I 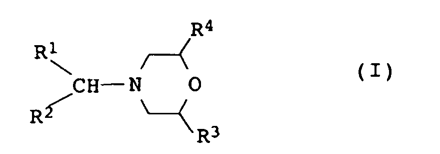 in which
R¹ and R², independently of one another, are hydrogen, alkyl or cycloalkyl, or R¹ and R² together with the carbon atom to which they are bonded are a 5- to 14-membered carbocycle, and
R³ and R⁴, independently of one another, are alkyl or cycloalkyl,
by reacting at least one carbonyl compound of the formula II 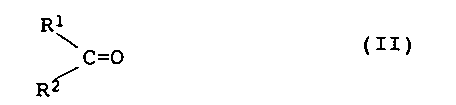 in which R¹ and R² have the meanings given above, with at least one morpholine of the formula III 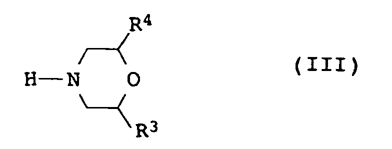 in which R³ and R⁴ have the meanings given above, in the presence of hydrogen and at least one metal-containing catalyst, wherein the active component of the catalyst consists essentially of platinum group metals.

2. The process according to claim 1, wherein a catalyst is used whose active component comprises
- 1 to 100% by weight of Pd,
- 0 to 60% by weight of Pt, and
- 0 to 50% by weight of at least one further metal, which is chosen from Ru, Rh, Os, Ir, Ce, La and mixtures thereof.

3. The process according to any of the preceding claims, wherein a catalyst is used which comprises a support.

4. The process according to claim 3, wherein the support used is ZrO₂.

5. The process according to any of the preceding claims, wherein a catalyst is used which comprises 0.1 to 10% by weight of Pd and 0 to 5% by weight of Pt.

6. The process according to any of the preceding claims, wherein R¹ and R² together with the carbon atom to which they are bonded are a 6- to 12-membered carbocycle which can have one, two or three substituents which are chosen, independently of one another, from alkyl, alkoxy, alkylthio, cycloalkyl and cycloalkylalkyl.

7. The process according to any of the preceding claims, wherein R³ and R⁴, independently of one another, are C₁-C₄-alkyl radicals.

8. The process according to any of the preceding claims, wherein R³ and R⁴ are both methyl.

9. The process according to any of the preceding claims, wherein the compound of the formula I is N-(cyclododecyl)-2,6-dimethylmorpholine.

## Revendications

1. Procédé de préparation de 2,6-dialkyl-morpholines N-substituées de la formule générale I : 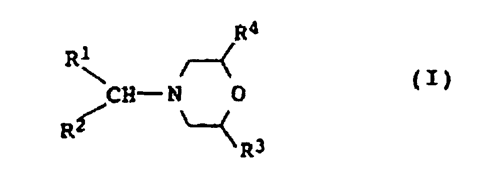 dans laquelle
R¹ et R² représentent indépendamment l'un de l'autre de l'hydrogène ou un groupe alkyle ou cycloalkyle, ou R¹ et R² forment ensemble avec l'atome de carbone sur lequel ils sont fixés un carbocycle de 5 à 14 termes, et
R³ et R⁴ représentent indépendamment l'un de l'autre un groupe alkyle ou cycloalkyle,
par réaction d'au moins un composé carbonyle de la formule générale II : 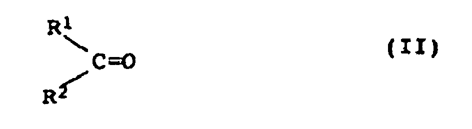 dans laquelle R¹ et R²ont les significations données ci-dessus, avec au moins une morpholine de la formule générale III : 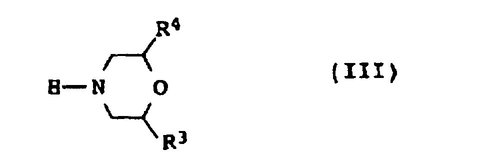 dans laquelle R³ et R⁴ ont les significations données ci-dessus, en présence d'hydrogène et d'au moins un catalyseur contenant du métal, **caractérisé en ce que** le composant actif du catalyseur est essentiellement constitué de métaux du groupe du platine.

2. Procédé suivant la revendication 1, **caractérisé en ce qu'**on met en oeuvre un catalyseur dont le composant actif contient
- 1 à 100 % en poids de Pd,
- 0 à 60 % en poids de Pt, et
- 0 à 50 % en poids d'au moins un autre métal qui est choisi parmi Ru, Rh, Os, Ir, Ce, La et leurs mélanges.

3. Procédé suivant l'une des revendications précédentes, **caractérisé en ce qu'**on met en oeuvre un catalyseur qui comporte un support.

4. Procédé suivant la revendication 3, **caractérisé en ce qu'**on met en oeuvre ZrO₂ comme support.

5. Procédé suivant l'une des revendications précédentes, **caractérisé en ce qu'**on met en oeuvre un catalyseur qui contient 0,1 à 10 % en poids de Pd et 0 à 5 % en poids de Pt.

6. Procédé suivant l'une des revendications précédentes, **caractérisé en ce que** R¹ et R² forment conjointement à l'atome de carbone sur lequel ils sont fixés un carbocycle de 6 à 12 termes qui peut présenter un, deux ou trois substituants qui sont choisis indépendamment l'un de l'autre parmi les groupes alkyle, alcoxy, alkylthio, cycloalkyle et cycloalkylalkyle.

7. Procédé suivant l'une des revendications précédentes, **caractérisé en ce que** R³ et R⁴ représentent indépendamment l'un de l'autre des radicaux alkyle en C₁-C₄.

8. Procédé suivant l'une des revendications précédentes, **caractérisé en ce que** R³ et R⁴ représentent tous deux du méthyle.

9. Procédé suivant l'une des revendications précédentes, **caractérisé en ce que**, en ce qui concerne le composé de la formule générale I, il s'agit de N-(cyclododécyl)-2,6-diméthylmorpholine.
